# FASCICULE DE BREVET EUROPEEN

(11) **EP 0 399 866 B1**
(45) Date de publication et mention de la délivrance du brevet: **22.02.1995**
(21) Numéro de dépôt: 90401224.2
(22) Date de dépôt: 09.05.1990
(51) Int. Cl.: C07C 251/88, C01B 21/16

(54) **Procédé de synthésé d'azines, son application à la production d'hydrazine**
Verfahren zur Synthese von Azinen, seine Verwendung zur Herstellung von Hydrazin
Process for the synthesis of azines, its application in the production of hydrazine

(30) Priorité: 24.05.1989 FR 8906803
(43) Date de publication de la demande: 28.11.1990
(73) Titulaire: ELF ATOCHEM S.A., 92800 Puteaux (FR)
(72) Inventeur: Schirmann, Jean-Pierre, F-69600 Oullins (FR); Pleuvry, Jean-Pierre, F-65250 La Barthe de Neste (FR); Tellier, Pierre, F-69110 Saint Foy Les Lyon (FR)

(56) Documents cités:
- EP-A- 0 179 699
- DE-A- 2 326 197
- FR-A- 2 120 517
- FR-A- 2 260 569
- CHEMICAL ABSTRACTS, vol. 100, 23 janvier 1984, page 73, résumé no. 24042z, Columbus, Ohio, US; & JP-A-58 120 507 (SHOWA DENKO K.K.) 18-07-1983

## Description

La présente invention concerne un procédé de synthèse d'azines et son application à la production d'hydrazine. L'hydrazine, le plus souvent sous la forme d'hydrate d'hydrazine est un produit très utilisé en tant que tel ou comme intermédiaire de synthèse. Selon KIRK OTHMER, 3° édition, volume 12, pages 734 à 755, l'hydrazine est produite soit par oxydation de l'ammoniac à l'aide de chlore ou d'eau de javel éventuellement en passant par une cetazine, soit par réaction de l'eau oxygénée d'ammoniac et d'une cétone.

La présente invention concerne le procédé à l'eau oxygénée ; il a été décrit dans de nombreux brevets, notamment US 3 972 878, US 3 972 876, US 3 869 541, US 3 948 902, US 3 919 256, US 3 943 152, US 4 093 656. Le KIRK OTHMER déjà cité décrit le principe d'une mise en oeuvre de ce procédé selon le brevet US 4 093 656. On fait réagir de l'eau oxygénée, de l'ammoniac et de la méthyléthylcétone en présence d'acétamide et de phosphate de sodium pour obtenir, après séparation, l'azine correspondante et une solution aqueuse (la solution de travail) contenant l'acétamide et le phosphate de sodium. Cette solution aqueuse est concentrée pour enlever l'eau de réaction ainsi que l'eau apportée par l'eau oxygénée, puis est recyclée au réacteur. L'azine est hydrolysée en hydrazine, on récupère la méthyléthylcétone qu'on recycle au réacteur (de synthèse d'azines).

Quand on opère en continu selon ce procédé, on constate une baisse d'activité de la solution de travail, c'est-à-dire une baisse de productivité du réacteur et il est nécessaire de refaire une nouvelle solution de travail. On trouve dans le brevet US 4 093 656 des exemples de différentes solutions de travail. On constate aussi dans l'art antérieur déjà cité que bien que le procédé de synthèse d'azines puisse fonctionner avec des réactifs en proportions très variables, on peut observer des variations sur le rendement en production d'azines et il est avantageux de maintenir certaines proportions notamment pour les produits contenus dans la solution de travail. FR 2 120 517 (=US 3 869 541) decrit un procedé de synthese d'azines à partir d'eau oxygenée, d'ammoniac, d'une cetone et d'un nitrile - le nitrile est transformé en amide (acetonitrile en acetamide). Il est necessaire ensuite de retransformer l'acetamide en acetonitrile. Cette reaction se fait vers 450°C pour avoir des vitesses significatives au cours d'un procedé à l'echelle industrielle.

On a maintenant trouvé un procédé beaucoup plus simple qui permet d'assurer une production continue et constante d'azines sans avoir à contrôler en détail la solution de travail.

La présente invention est donc un procédé de synthèse d'azines à partir d'eau oxygénée, d'ammoniac et d'un réactif portant un groupe carbonyle, caractérisé en ce que :
a) on met en contact ces réactifs avec une solution, dite solution de travail, jusqu'à obtenir de l'azine,
b) on sépare (i) l'azine et éventuellement l'excès de réactif portant un groupe carbonyle et (ii) la solution de travail,
c) on porte tout ou partie de la solution de travail à une temperature entre 150 et 250°C de preference entre 170 et 230°C, l'eau de réaction étant éliminée avant, après ou simultanément,
d) on recycle la solution de travail à l'étape a),
e) on introduit dans la solution de travail en un point quelconque des étapes précédentes un produit capable d'entretenir la production d'azine.

La réaction peut être représentée par l'équation suivante :

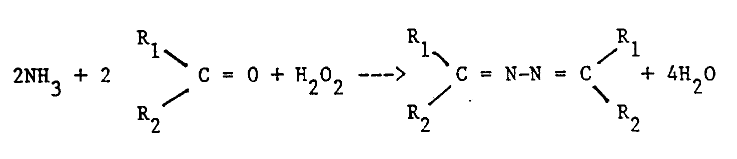

L'eau oxygénée peut être utilisée sous la forme commerciale habituelle, par exemple en solution aqueuse entre 30 et 90 % en poids de H₂O₂. Avantageusement, on peut ajouter un ou plusieurs stabilisants usuels des solutions péroxydiques, par exemple de l'acide phosphorique, pyrophosphorique, citrique, nitrilotriacétique, éthylènediaminetétracétique ou les sels d'ammonium ou de métal alcalin de ces acides. La quantité à utiliser est avantageusement comprise entre 10 et 1000 ppm et, de préférence, entre 50 et 250 ppm de l'ensemble des réactifs et de la solution de travail à l'entrée du réacteur. L'ammoniac peut être anhydre ou en solution aqueuse. Ce réactif portant un groupe carbonyl est de formule : 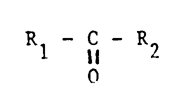
dans laquelle R₁ et R₂, identiques ou différents, représentent l'hydrogène, un radical alkyl ayant de 1 à 12 atomes de carbone, un radical alkyl ramifié ou cycloalkyl ayant de 3 à 12 atomes de carbone, un radical aromatique ayant de 6 à 12 atomes de carbone ou représentent ensemble un radical alkylène linéaire ou ramifié ayant de 3 à 12 atomes de carbone ; ces radicaux pouvant être substitués par un halogène, un groupe NO₂, hydroxy, alkoxy ou ester carboxylique, et de préférence Cl, NO₂ ou CH₃O.

Des exemples de réactifs : 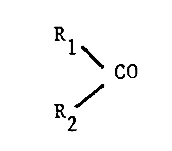
sont des aldéhydes ou des cétones.

Parmi les aldéhydes on peut citer le formaldéhyde, l'acétaldéhyde, le butyraldéhyde, l'isobutyraldéhyde, le n-pentanal, le pivalaldéhyde, le benzaldéhyde, les monochlorobenzaldéhydes, le paranitrobenzaldéhyde, l'anisaldéhyde, le beta-chloropropionaldéhyde, le beta-méthoxypropionaldéhyde.

Parmi les cétones, on peut citer l'acétone, la 2-butanone, la 2-pentanone, la 3-pentanone, la méthyl isopropyl cétone, la méthyl isobutyl cétone, la méthyl éthyl cétone, la méthyl cyclohexyl cétone, l'acétophénone, la benzophénone, la cyclobutanone, la cyclopentanone, la cyclohexanone.

On utilise avantageusement les cétones dans lesquelles R₁ et R₂ sont identiques ou différents, et sont des alkyls linéaires ou ramifiés ayant de 1 à 5 atomes de carbone et de préférence l'acétone, la méthyléthyl cétone et la méthyl isobutyl cétone.

L'azine peut être hydrolysée en hydrate d'hydrazine selon l'équation : 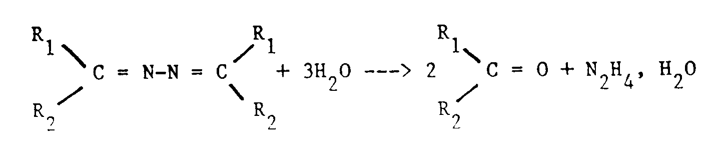
en régénérant le réactif : 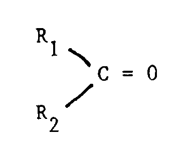
qui est recyclé au réacteur. On ne sortirait pas du cadre de l'invention en utilisant des mélanges d'aldéhydes, d'aldéhydes et de cétones ou de cétones. Par exemple, en utilisant un mélange de : 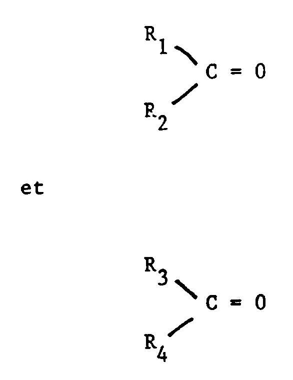
R₃ et R₄ ayant la même signification que R₁ et R₂, ledit mélange pouvant aussi contenir : 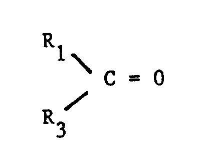
on obtient des mélanges d'azines symétriques et dissymétriques du genre : 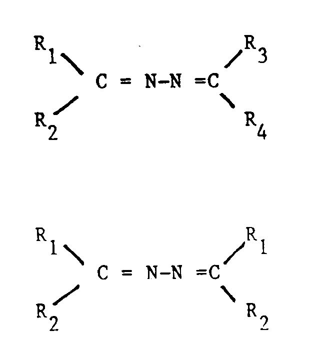 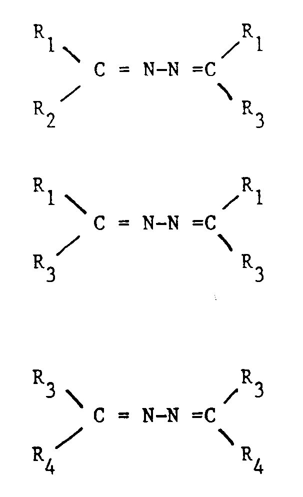
Comme précédemment par l'hydrolyse, on régénère le mélange des cétones ou aldéhydes.

La réaction d'hydrolyse des cetazines est décrite dans les brevets US 4 724 133 et US 4 725 421.

Les réactifs peuvent être utilisés en quantités stoéchiométriques, cependant on utilise par mole d'eau oxygénée 0,2 à 5 moles et de préférence 1,5 à 4 moles du réactif carboxylé (aldéhyde ou cétone) ; 0,1 à 10 moles et de préférence 1,5 à 4 moles d'ammoniac. La quantité de solution de travail est comprise entre 0,1 et 1 kg par mole d'eau oxygénée. La qualité de la solution de travail, c'est-à-dire sa force catalytique ou son activité qui permet de convertir les réactifs en azine dépend du traitement de l'étape c et du produit de l'étape e. Les proportions des réactifs fixées ci-dessus permettent d'obtenir une conversion totale de l'eau oxygénée et une production d'azine correspondant à plus de 50 % de l'eau oxygénée engagée et pouvant atteindre 90 %.

La solution de travail est une solution quelconque pourvu qu'elle puisse contenir l'eau de réaction et l'emmener jusqu'à l'étape c. On ne connait pas la composition de cette solution et c'est justement l'avantage de l'invention par rapport à l'art antérieur. On forme une solution de travail initiale pour démarrer l'installation de production, puis après démarrage de la production, le traitement de l'étape c et l'introduction du produit de l'étape e, font que la solution de travail est apte à la production d'azines. On surveille le volume de la solution de travail qu'on peut ajuster en éliminant plus ou moins d'eau en c. On surveille son activité en mesurant la production d'azine.

On décrira la formation de la solution de travail initiale lors de la description de e.

La mise en contact de l'eau oxygénée, de l'ammoniac, du réactif portant un groupe carbonyl avec la solution de travail peut s'effectuer de façon quelconque.

Avantageusement on opère dans un milieu homogène ou dans un milieu qui assure au moins une solubilisation suffisante des réactifs pour pouvoir obtenir de l'azine. La réaction peut se faire dans une très large plage de température, par exemple entre 0 et 100°C, et on opère avantageusement entre 30 et 70°C. Bien qu'on puisse opérer à toute pression, il est plus simple d'être à la prèssion atmosphérique, mais on peut monter jusqu'à environ 10 bars si c'est nécessaire pour maintenir de préférence la réaction de l'étape a en phase liquide.

Les réactifs peuvent être introduits simultanément ou séparément et dans un ordre quelconque dans la solution de travail. On peut utiliser toutes sortes de réacteurs, agités ou non agités, ou même de simples capacités qu'on peut disposer en parallèle en série à co-courant ou à contre-courant, ou toute combinaison de ces possibilités.

On sépare (i) l'azine et éventuellement l'excès de réactif portant un groupe carbonyl et (ii) la solution de travail par des moyens connus tels que l'extraction liquide-liquide, la distillation ou toute combinaison de ces possibilités.

L'azine et l'excès de réactif portant un groupe carbonyl peuvent former une phase homogène ou n'être que partiellement miscibles. Il est avantageux de les séparer avant d'hydrolyser l'azine. Cet excès de réactif carbonylé et celui qu'on recueille après l'hydrolyse de l'azine peuvent être recyclés au réacteur. Si l'ammoniac est en excès dans l'étape a, il est entraîné avec l'eau de réaction dans la solution de travail.

Le traitement de l'étape c consiste sur tout ou partie de la solution de travail (i) à éliminer l'eau de réaction et (ii) à porter à une température entre 150 et 250°C; (i) et (ii) pouvant être dans un ordre quelconque ou simultanées.

L'élimination d'eau est une opération ordinaire qui peut être avantageusement faite dans une colonne à distiller en prenant soin de récupérer les produits plus volatils que l'eau. Parmi les produits plus volatils que l'eau qui sortent, on trouve de l'ammoniac, un peu de réactif portant un groupe carbonyl si on a utilisé dans l'étape a un tel produit plus léger que l'eau, du méthanol ou de l'éthanol si la solution de travail en contient. L'ammoniac, le composé carbonylé éventuel sont retournés au réacteur. Le méthanol ou l'éthanol après séparation d'avec l'eau sont recyclée à l'étape a au réacteur ou mélangés avec la solution de travail en sortie de l'étape c. Le traitement au-delà de 130°C peut se faire dans un échangeur, ou un rebouilleur de fond de colonne.

Avantageusement, la solution de travail est portée à une température entre 150 et 250°C en éliminant simultanément l'eau de réaction. Avantageusement la solution de travail est portée à une température comprise de préférence entre 170 et 230°C. Cette opération peut s'effectuer de nombreuses manières, il est avantageux d'opérer dans une capacité dotée d'un système de chauffage, ladite capacité étant surmontée d'un moyen de distillation pouvant être une colonne à distiller, ou tout dispositif ayant la même fonction. L'ensemble capacité/colonne à distiller est alimenté par la solution de travail dans la colonne à distiller. Selon une forme préférée de l'invention, le dispositif de cette étape c est une colonne à distiller, le fond de cette colonne (la partie basse) est maintenu à au moins 130°C, et cette colonne est alimentée latéralement par la solution de travail. La pression de cette colonne est ajustée par des moyens usuels tels que le débit de réfrigérant sur le condenseur de reflux ou le système de vide pour assurer une ébullition en pied et on classe la colonne pour avoir en tête l'eau et les produits plus volatils que l'eau. Le débit de soutirage d'eau en tête se fait pour assurer un volume constant de la solution de travail. Parmi les produits plus volatils que l'eau qui sortent, on trouve de l'ammoniac, un peu de réactif portant un groupe carbonyl si on a utilisé dans l'étape a un tel produit plus léger que l'eau, du méthanol ou de l'éthanol si la solution de travail en contient. L'ammoniac, le composé carbonylé éventuel sont retournés au réacteur. Le méthanol ou l'éthanol après séparation d'avec l'eau sont recyclée à l'étape a au réacteur ou mélangés avec la solution de travail en sortie de l'étape c. Selon une forme préférée de l'invention, la colonne comprend de 5 à 50 plateaux théoriques et l'alimentation se fait entre le 3° et le 20° plateau comptés en partant du fonds de la colonne.

Dans la forme préférée de l'invention qui est une colonne à distiller, la température de la solution de travail qui alimente la colonne est inférieure à 110°C, la température du fonds de la colonne est comprise entre 150 et 250°C et le temps de séjour compris entre 10 minutes et 2 heures. Dans cette dernière forme préférée la température d'alimentation est comprise entre 50 et 110°C, la température du fonds de colonne est comprise entre 170 et 230°C et le temps de séjour entre 10 minutes et quelques heures.

Il n'est pas nécessaire de traiter toute la solution de travail dans l'étape c, on peut en fin d'étape b traiter seulement une partie de la solution puis mélanger la partie de la solution traitée en c et la partie non traitée et retourner l'ensemble au réacteur de l'étape a.

Avantageusement la partie qu'on traite représente au moins 50 % en poids de l'ensemble de la solution de travail et de préférence 60 à 100 % selon l'activité de cette solution nécessaire pour la production d'azines.

L'avantage du procédé de la présente invention est qu'on remplit l'installation avec une solution de travail initiale, puis qu'on la fait tourner en la traitant à l'étape c et en y introduisant un produit capable d'entretenir la production d'azine. Le produit de l'étape e peut être choisi parmi les oxyacides organiques ou inorganiques, leurs sels d'ammonium et d'une façon générale leurs dérivés : anhydrides, esters, amides, nitriles, péroxydes d'acyles, ou leur mélange. Avantageusement on utilise les amides, les sels d'ammonium et les nitriles.

A titre d'exemple, on peut citer (i) des amides d'acides carboxyliques de formule R₅COOH dans laquelle R₅ est l'hydrogène, un radical alkyl linéaire ayant de 1 à 20 atomes de carbone, ou un radical alkyl ramifié ou cyclique ayant de 3 à 12 atomes de carbone, ou un radical phényl pouvant être substitué, (ii) des amides d'acides polycarboxyliques de formule R₆(COOH)ₙ dans laquelle R₆ représente un radical alkylène ayant de 1 à 10 atomes de carbone et n valant 1 ou 2, R₆ peut être une simple liaison alors n vaut 2. Les radicaux R₅ et R₆ peuvent être substitués par des halogènes, des groupements OH, NO₂ ou méthoxy. On peut citer aussi les amides des acides organiques de l'arsenic.

Les amides préférés sont le formamide, l'acétamide, le monochloracétamide et le propionamide.

Parmi les sels d'ammonium on utilise avantageusement les sels d'hydracides, d'oxyacides minéraux d'acides arylsulphoniques, d'acides R₅COOH ou d'acides R₆(COOH)ₙ, R₅, R₆ et n étant définis précédemment, des acides organiques de l'arsenic.

Les sels d'ammonium préférés sont le formiate, l'acétate, le monochloracétate, le propionate, le phénylarsonate et le cacodylate. Parmi les nitriles, on peut citer avantageusement les produits de formule R₇(CN)ₙ, n pouvant varier de 1 à 5 selon la valence de R₇, R₇ est un alkyl cyclique ou non cyclique ayant de 1 à 12 atomes de carbone ou de benzène ou de la pyridine. R₇ peut être substitué par des groupements qui ne s'oxydent pas dans le réacteur de l'étape a, par exemple des halogènes, des groupes carboxyliques, esters carboxyliques, nitro, amine, hydroxy ou acide sulphonic.

Les nitriles préférés sont l'acétonitrile et le propionitrile.

On forme la solution de travail en mettant en solution un ou plusieurs produits choisis parmi les oxyacides organiques ou inorganiques, leurs sels d'ammonium et d'une façon générale leurs dérivés : anhydrides, esters, amides, nitriles, péroxydes d'acyles, ou leur mélange. Avantageusement on utilise les amides, les sels d'ammonium ou les nitriles précédents.

Cette solution peut être aqueuse ou à base d'un alcool ou d'un mélange d'alcool et eau. Parmi les alcools on utilise avantageusement les alcools aliphatiques saturés ayant de 1 à 6 atomes de carbone et de préférence 1 à 2 atomes de carbone.

On utilise aussi avantageusement des diols et plus particulièrement des diols ayant de 2 à 5 atomes de carbone. On peut citer par exemple le glycol, le propylèneglycol, le 1,3 propanediol, le 1,3 et 1,4 butanediol et le 1,5 pentanediol.

On démarre le cycle de cette solution de travail entre le réacteur en a, la séparation en b, le traitement en c et le retour en a. On opère ainsi "à vide", c'est-à-dire sans production d'azine, puis on démarre progressivement l'introduction des réactifs dans le réacteur de l'étape a. Dès que la production d'azine est en régime, on introduit en continu un produit capable d'entretenir la production d'azine et choisi avantageusement parmi les amides, les sels d'ammonium ou les nitriles.

Que ce soit pour former la solution de travail initiale ou pour l'appoint de l'étape e, on ne sortirait pas du cadre de l'invention en formant in situ le sel d'ammonium en profitant de l'ammoniac, il suffit d'utiliser l'acide carboxylique correspondant. Par exemple on peut remplacer un appoint d'acétate d'ammonium par un appoint d'acide acétique, de même pour constituer la solution de travail initiale, il suffit d'opérer "à vide", c'est-à-dire sans l'eau oxygénée mais avec de l'ammoniac. Puis, comme précédemment, on fait tourner cette solution de travail en formation et après on démarre la production en ajoutant les réactifs, c'est-à-dire l'ammoniac, l'eau oxygénée et le composé carbonylé. La quantité d'amide, d'acide, de sels d'ammonium ou de nitrile à mettre dans l'eau, l'alcool ou le mélange d'eau et d'alcool est comprise entre 40 et 80 % en poids de la solution de travail à l'entrée de l'étape a.

Cette quantité est exprimée en équivalent acide acétique, c'est-à-dire qu'une mole d'acétate d'ammonium ou une mole de propionamide ou une mole d'acétonitrile ou une mole de formamide, sont équivalentes à une mole d'acide acétique. On peut ne mettre que de l'amide, que des sels d'ammonium ou que du nitrile, ou un mélange de deux ou de ces trois produits, sachant que l'un d'eux peut être lui-même un mélange (de plusieurs amides, de plusieurs sels d'ammonium ou de plusieurs nitriles).

Avantageusement, on forme la solution de travail initiale en diluant le sel d'ammonium dans l'eau à raison d'environ 50 % en poids et de préférence on le forme in situ à partir d'acide acétique. Quant à l'introduction du produit de l'étape e, on peut utiliser tout produit ou tout mélange d'amides de sels d'ammonium ou de nitriles précédents. On peut aussi utiliser tout produit formant le sel d'ammonium in situ, par exemple l'acide acétique. La quantité est ajustée en fonction de la production d'azine. C'est l'homme de métier qui peut déterminer facilement la quantité à ajouter pour entretenir la production d'azine. On ne connaît pas le mécanisme de cette synthèse ni la contribution de l'étape c et de l'étape e du procédé de l'invention dans la production d'azine.

On observe généralement qu'il suffit d'au plus 1 % en poids rapporté à la solution de travail à l'entrée de a.

Il n'y a pas de relation entre le produit qui sert à former la solution de travail initiale et le produit qu'on introduit dans l'étape e, on peut utiliser toute combinaison. Avantageusement on constitue la solution de travail avec un sel d'ammonium formé in situ ou un nitrile et le produit de l'étape e est un nitrile ou un acide carboxylique R₅COOH ou R₆(COOH)ₙ. De préférence on forme la solution de travail avec de l'acide acétique et de l'ammoniac et on introduit dans l'étape e de l'acide acétique.

Selon une forme préférée de l'invention, on peut éliminer certaines impuretés à haut point d'ébullition de la solution de travail. Il suffit de traiter la solution de travail en totalité ou en partie et avantageusement on purifie la partie de la solution de travail qui sort de l'étape c. L'élimination de ces impuretés à haut point d'ébullition peut se faire tout simplement par distillation en rejetant en tout ou partie tout ce qui bout au-dessus du composant (ou ses sels) servant à faire la solution de travail ou à entretenir son activité (ou du composé plus lourd en cas de mélange). Cette quantité d'impuretés représente entre 0,01 et 5 % en poids de la totalité de la solution de travail.

Cette quantité est faible et peut dépendre des conditions de la synthèse d'azines, des impuretés déjà présentes dans l'ammoniac, l'eau oxygénée et le composé carboxylé, et on sait que l'eau oxygénée en présence d'impuretés peut conduire à des impuretés lourdes.

Au lieu de procéder par distillation, on peut aussi éliminer ces impuretés de la solution de travail par passage sur des matières absorbantes constituées de particules microporeuses. On peut utiliser par exemple les produits décrits dans le brevet US 4 657 751.

Selon une forme préférée de l'invention, on peut ajouter dans la solution de travail entre l'étape c et l'étape a ou dans la réaction de l'étape a, un catalyseur permettant de stabiliser la réaction de synthèse d'azines. Ce catalyseur a la formule générale Q-X-Y = Z, dans laquelle Q est l'hydrogène ou un métal alcalin ou l'ammonium ; X et Z sont des atomes d'oxygène ou d'azote et Y est un atome de carbone, d'azote, d'arsenic, d'antimoine, de phosphore, de soufre, selenium ou tellure ; les atomes X, Y et Z portent les substituants nécessaires pour satisfaire les règles de valence. Ce catalyseur Q-X-Y = Z peut être un phosphate, phosphite, phosphonate, polyphosphate, pyrophosphate, arsenate, un phénylarsenate, un cacodylate, bicarbonate, antimonate, stannate ou un sulfate d'ammonium ou de métaux alcalins. On peut utiliser aussi les esters correspondants, en particulier ceux des alcools aliphatiques ayant de 1 à 5 atomes de carbone. La quantité à utiliser est avantageusement comprise entre 10 et 1000 ppm, et de préférence, entre 50 et 250 ppm de l'ensemble solution de travail, réactifs à l'entrée de a. Les phosphates de métaux alcalins et l'orthophosphate disodique sont préférés.

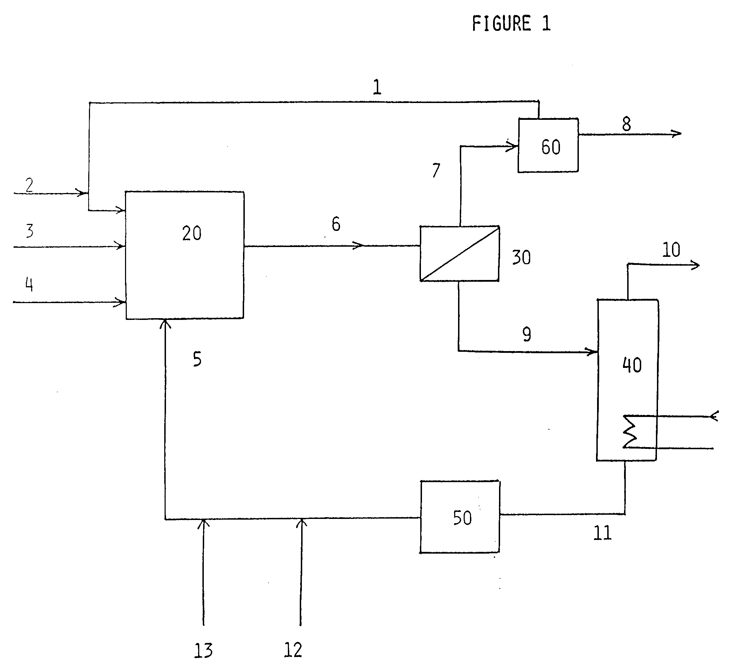

La figure 1 ci-jointe est un mode de réalisation de la présente invention. 20 représente le réacteur de l'étape a, 30 le décanteur séparant l'azine de la solution de travail, 60 le dispositif d'hydrolyse de l'azine, 40 la colonne assurant l'étape c et 50 le dispositif avec la résine échangeuse d'ions qui élimine les impuretés lourdes. Pour simplifier, on n'a pas représenté les pompes, les vannes et le reflux de la colonne 40.

1 est le recyclage de méthyl éthyl cétone (MEK), 2 l'appoint de MEK, 3 l'alimentation en eau oxygénée, 4 l'ammoniac et 5 la solution de travail. On sort en 6 un mélange d'azine et de solution de travail qu'on sépare dans le décanteur 30. L'azine est transférée en 7 vers l'hydrolyse pour produire en 8 de l'hydrate d'hydrazine, la MEK étant recyclée par 1 au réacteur. En 9 la solution de travail entre dans la colonne 40 d'où on récupère en 10 de l'eau et un peu d'ammoniac. Le pied 11 passe sur des résines absorbantes en polystyrène de porosité en volume 51 %, de surface 750 m²/g, dont le diamètre moyen des pores est 50 Å et la granulométrie 1,2 à 0,3 mm (20 à 50 mesh). Puis retourne en 20 par l'entrée 5. En 12 on ajoute de l'acide acétique selon l'étape e du procédé et en 13 du phosphate disodique.

### EXEMPLE 1

On opère selon le dispositif de la figure 1 sauf que 30 % en poids de la solution de travail en 9 n'est pas traité en 40 et rejoint la partie traitée après 50. On a formé une solution de travail en mélangeant de l'eau, de l'acide acétique et de l'ammoniac, tout en chauffant progressivement la colonne 40. La quantité d'acide acétique utilisée pour faire de l'acétate d'ammonium représente environ 50 % en poids de la solution de travail. On a obtenu en régime stationnaire :
En 2 appoint de 100 kg/h de méthyl éthyl cétone (MEK),
En 3, 1000 kg/h d'H₂O₂ stabilisé a l'EDTA, exprimé en H₂O₂ 100 % (sous forme d'une solution à 70 %),
En 4, 1000 kg/h NH₃,
En 13, 3 kg/h de phosphate disodique,
En 12, 60 kg/h d'acide acétique,
Le réacteur 20 opère à 50°C,
En 5, le débit de solution de travail est de 11000 kg/h,
En 7, on obtient un débit de 3900 kg/h de mékazine :

CH₃(C₂H₅)C=N-N=C(C₂H₅)CH₃

En 9, la solution de travail à 50°C alimente la colonne 40 au 11° plateau au dessus du bouilleur, il y a 19 plateaux entre l'alimentation et la tête. La pression de tête de 40 est 0,9 bar absolus, le pied est à 180°C, le bouilleur est alimenté par 10500 kg/h de vapeur 18 bars absolus.

### EXAMPLE 2

On opère comme dans l'exemple 1, mais en utilisant du formiate d'ammonium au lieu d'acétate d'ammonium et en faisant passer la solution de travail sortant de 30 dans une capacité lui asssurant un chauffage à 200°C avant de l'envoyer dans la colonne 40. Cette capacité est chauffée en série avec le bouilleur de 40, le débit de vapeur sur cette capacité et sur le bouilleur 40 est aussi de 10500 Kg/h.

Le formiate est formé in situ par l'ajout d'acide formique.

On remplace en 12 60 Kg/h d'acide acétique par 53 Kg/h d'acide formique. Les autres paramètres sont identiques.

## Revendications

1. Procédé de synthèse d'azines à partir d'eau oxygénée, d'ammoniac et d'un réactif portant un groupe carbonyl, caractérisé en ce que :
a) on met en contact ces réactifs avec une solution, dite solution de travail, jusqu'à obtenir de l'azine,
b) on sépare (i) l'azine et éventuellement l'excès de réactif portant un groupe carbonyl et (ii) la solution de travail,
c) on porte tout ou partie de la solution de travail à une temperature entre 150 et 250°C de preference entre 170 et 230°C, l'eau de réaction étant éliminée avant, après ou simultanément,
d) on recycle la solution de travail à l'étape a),
e) on introduit dans la solution de travail en un point quelconque des étapes précédentes un produit capable d'entretenir la production d'azine.

2. Procédé selon la revendication 1, caractérisé en ce que le réactif portant un groupe carbonyle est choisi parmi l'acétone, la méthyl éthyl cétone et la méthyl isobutyl cétone.

3. Procédé selon la revendication 1 ou 2, caractérisé en ce que l'azine est hydrolysée en hydrate d'hydrazine.

4. Procédé selon l'une des revendications 1 à 3, caractérisé en ce que dans l'étape a la quantité de solution de travail est comprise entre 0,1 et 1 kg par mole d'eau oxygénée.

5. Procédé selon l'une des revendications 1 à 4, caractérisé en ce qu'on effectue l'étape a entre 30 et 70°C.

6. Procédé selon l'une des revendications 1 à 5 caractérisé en ce que dans l'étape c l'eau est éliminée simultanément et en ce que l'étape c est effectuée dans une colonne de distillation alimentée latéralement par la solution de travail et dont le pied et à une température supérieure à 130°C.

7. Procédé selon l'une des revendication 1 à 6, caractérisé en ce que le produit de l'étape e est choisi parmi les amides, les sels d'ammonium ou les nitriles.

8. Procédé selon la revendication 7, caractérisé en ce que les sels d'ammonium peuvent être formés in situ.

9. Procédé selon la revendication 8, caractérisé en ce que le produit introduit est de l'acide acétique.

10. Procédé selon la revendication 8, caractérisé en ce que le produit introduit est l'acide phénylarsonique.

11. Procédé selon la revendication 8, caractérisé en ce que le produit introduit est l'acide cacodylique.

12. Procédé selon l'une des revendications 1 à 11, caractérisé en ce que la solution de travail est purifiée par élimination des impuretés à haut point d'ébullition.

13. Procédé selon la revendication 12, caractérisé en ce que la solution de travail est purifiée par passage sur des résines.

14. Procédé selon l'une des revendications 1 à 13, caractérisé en ce qu'on ajoute dans la solution de travail entre l'étape c et l'étape a ou dans le réacteur de l'étape a, un catalyseur de formule générale Q-X-Y = Z dans laquelle Q est l'hydrogène ou un métal alcalin ou l'ammonium ; X et Z sont des atomes d'oxygène ou d'azote et Y est un atome de carbone, d'azote, d'arsenic, d'antimoine, de phosphore, de soufre, selerium ou tellure ; les atomes X, Y et Z portent les substituants nécessaires pour satisfaire les règles de valence.

## Claims

1. Process for the synthesis of azines from hydrogen peroxide, ammonia and a reagent having a carbonyl group, characterized in that:
a) these reagents are put in contact with a solution, called the working solution, until the azine is obtained,
b) (i) the azine and optionally the excess of reagent having a carbonyl group is separated from (ii) the working solution,
c) all or a portion of the working solution is raised to a temperature between 150 and 250°C, preferably between 170 and 230°C, the water produced by the reaction being eliminated before, after or simultaneously,
d) the working solution is recycled to step a),
e) a compound capable of maintaining the production of azine is introduced into the working solution at any point in the preceding steps.

2. Process according to Claim 1, characterized in that the reagent having a carbonyl group is chosen from acetone, methyl ethyl ketone and methyl isobutyl ketone.

3. Process according to Claim 1 or 2, characterized in that the azine is hydrolysed to hydrazine hydrate.

4. Process according to one of Claims 1 to 3, characterized in that, in step a, the quantity of working solution is between 0.1 and 1 kg per mole of hydrogen peroxide.

5. Process according to one of Claims 1 to 4, characterized in that step a is carried out between 30 and 70°C.

6. Process according to one of Claims 1 to 5, characterized in that, in step c, the water is eliminated simultaneously, and in that step c is carried out in a distillatlon column which is fed from the side with the working solution and the foot of which is at a temperature greater than 130°C.

7. Process according to one of Claims 1 to 6, characterized in that the compound of step e is chosen from amides, ammonium salts or nitriles.

8. Process according to Claim 7, characterized in that the ammonium salts may he formed in situ.

9. Process according to Claim 8, characterized in that the compound introduced is acetic acid.

10. Process according to Claim 8, characterized in that the compound introduced is phenylarsonic acid.

11. Process according to Claim 8, characterized in that the compound introduced is cacodylic acid.

12. Process according to one of Claims 1 to 11, characterized in that the working solution is purified by elimination of high-boiling-point impurities.

13. Process according to Claim 12, characterized in that the working solution is purified by being passed over resins.

14. Process according to one of Claims 1 to 13, characterized in that a catalyst of general formula Q-X-Y=Z, in which Q is hydrogen or an alkali metal or ammonia; X and Z are oxygen or nitrogen atoms and Y is a carbon, nitrogen, arsenic, antimony, phosphorus, sulphur, selenium or tellurium atom; atoms X, Y and Z carry the necessary substituents to satisfy the valency rules, is added to the working solution between step c and step a, or to the reactor of step a.

## Patentansprüche

1. Verfahren zur Herstellung von Azinen ausgehend von Wasserstoffperoxid, Ammoniak und einem eine Carbonyl-Gruppe tragenden Reagenz, dadurch gekennzeichnet, daß
a) man die Reagenzien mit einer Lösung, genannt Arbeitslösung, in Kontakt bringt, bis man das Azin erhält,
b) man (i) das Azin und gegebenenfalls den Überschuß an dem eine Carbonyl-Gruppe tragenden Reagenz und (ii) die Arbeitslösung trennt,
c) man die Arbeitslösung ganz oder teilweise auf eine Temperatur zwischen 150 und 250 °C, vorzugsweise zwischen 170 und 230 °C, bringt; das Reaktionswasser wird vorher, nachher oder gleichzeitig entfernt,
d) man die Arbeitslösung des Schritts a) rezykliert,
e) man zu der Arbeitslösung zu irgendeinem Punkt der vorhergehenden Schritte ein Produkt hinzugibt, das das Entstehen von Azin aufrechterhalten kann.

2. Verfahren nach Anspruch 1, dadurch gekennzeichnet, daß das eine Carbonyl-Gruppe tragende Reagenz ausgewählt ist aus Aceton, Methylethylketon und Isobutylmethylketon.

3. Verfahren nach Anspruch 1 oder 2, dadurch gekennzeichnet, daß das Azin als Hydrazinhydrat hydrolysiert ist.

4. Verfahren nach einen, der Ansprüche 1 bis 3, dadurch gekennzeichnet, daß in der Schritt a) die Menge der Arbeitslösung zwischen 0,1 und 1 kg pro mol Wasserstoffperoxid liegt.

5. Verfahren nach einem der Ansprüche 1 bis 4, dadurch gekennzeichnet, daß man den Schritt a) bei einer Temperatur zwischen 30 und 70 °C durchführt.

6. Verfahren nach einem der Ansprüche 1 bis 5, dadurch gekennzeichnet, daß im Schritt c) das Wasser gleichzeitig entfernt wird und daß der Schritt c) in einer Destillationskolonne durchgeführt wird, die durch die Arbeitslösung seitlich gespeist wird, und daß der Sumpf eine Temperatur von mehr als 130 °C hat.

7. Verfahren nach einem der Ansprüche 1 bis 6, dadurch gekennzeichnet, daß das Produkts des Schritts e) ausgewählt ist aus den Amiden, Ammoniumsalzen oder Nitrilen.

8. Verfahren nach Anspruch 7, dadurch gekennzeichnet, daß die Ammoniumsalze in situ gebildet werden.

9. Verfahren nach Anspruch 8, dadurch gekennzeichnet, daß das hinzugegebene Produkt Essigsäure ist.

10. Verfahren nach Anspruch 8, dadurch gekennzeichnet, daß das hinzugegebene Produkt Phenylarsonsäure ist.

11. Verfahren nach Anspruch 8, dadurch gekennzeichnet, daß das hinzugegebene Produkt Kakodylsäure ist.

12. Verfahren nach einem der Ansprüche 1 bis 11, dadurch gekennzeichnet, daß die Arbeitslösung durch Entfernung der Verunreinigungen bei hohem Siedepunkt gereinigt wird.

13. Verfahren nach Anspruch 12, dadurch gekennzeichnet, daß die Arbeitslösung durch Überleiten über Harze gereinigt wird.

14. Verfahren nach einem der Ansprüche 1 bis 13, dadurch gekennzeichnet, daß man zu der Arbeitslösung zwischen dem Schritt c) und Schritt a) oder in den Reaktor des Schritts a) einen Katalysator der allgemeinen Formel Q-X-Y=Z gibt, in dieser Formel ist Q ein Wasserstoff-, ein Alkalimetallatom oder eine Ammoniumgruppe; X und Z sind Sauerstoff- oder Stickstoffatome und Y ist ein Kohlenstoff-, Stickstoff-, Arsen-, Antimon-, Phosphor-, Schwefel-, Selen- oder Telluratom; die Atome X, Y und Z tragen die zur Sättigung nach der Valenzregel notwendigen Substituenten.
